# EUROPEAN PATENT APPLICATION

(11) **EP 2 469 451 A2**
(43) Date of publication of application: **27.06.2012**
(21) Application number: 11180624.6
(22) Date of filing: 08.09.2011
(51) Int. Cl.: G06K 9/00, A61B 5/00, G02B 21/00

(54) **Biological information measuring apparatus**

(30) Priority: 08.09.2010 JP 2010200535
(71) Applicant: Yokogawa Electric Corporation, Tokyo 180-8750 (JP)
(72) Inventor: Tezuka, Shinichiro, Tokyo, 180-8750 (JP); Hara, Hitoshi, Tokyo, 180-8750 (JP)
(74) Representative: Henkel, Breuer & Partner

(57) **Abstract**

Biological information measuring apparatus capable of automatically identifying a subject under test, and executing continuous measurement under a common measurement condition having selected a properly identified site at a high S/N ratio for relatively long hours as necessary, and also improving utilization efficiency of the output light of a laser. In the biological information measuring apparatus using a confocal optical system provided with a laser serving as a light source thereof, the biological information measuring apparatus comprises a laser drive means (14) for effecting AC-driving so as to cause pulsed light to be outputted from the laser, and wherein the skin of a finger (FG) is discriminated from a nail (a) thereof on the basis of a detection signal of the confocal optical system, and a portion of the skin, in a range of the nail epithelium (b) of the finger (FG) to the first joint (c) thereof, is indicated as a measurement region.

## Description

### BACKGROUND

### Technical Field

The present invention relates to a biological information measuring apparatus, and in particular, to a biological information measuring apparatus using a confocal optical system.

### Related Art

When a subject under test is irradiated with light from a light source by making use of near-infrared spectroscopy, and so forth, light absorption characteristics according to an amount of a component of the subject under test is indicated in a wavelength range unique to the component contained in the subject under test, and therefore, techniques have since been known whereby absorbance (light absorption capacity of a specific component) is worked out from measurement light such as reflected light from the subject under test, and so forth to thereby analyze components contained in the subject under test on the basis of a absorbance spectrum of the measurement light.

As a biological information measuring apparatus for measuring components of the internal tissues of a biological object {for example, various substances (concentration of a component such as a blood glucose value in blood, and so forth) contained in blood of a blood vessel of, for example, a human body · an animal, or in a tissue fluid of the tissues thereof}, in particular, there has been proposed a biological information measuring apparatus for measuring respective reflected light beams by measuring respective laser beams at not less than two wavelengths, outgoing from a wavelength tunable laser, and reflected by the internal tissues of a biological object as a subject under test, and light transmitted through a biological object with the use of the wavelength tunable laser, and a confocal optical system, as is the case with Patent Document 1, thereby measuring components of the internal tissues of the biological object.

More specifically, the respective laser beams at not less than two wavelengths are irradiated against the internal tissues of the biological object as the subject under test, components of the internal tissues of the biological object are worked out by conducting a well-known test after an absorbance spectrum is found from light reflected by the internal tissues of the biological object, and light transmitted through the biological object, and a formula for expressing correlation between the absorbance spectrum, and the components of the internal tissues of the biological object (hereinafter referred to as correlation formula) is prepared to be kept in memory in advance, and absorbance worked out from the reflected light from the internal tissues of the biological object, and the light transmitted through the biological object are substituted for those in the correlation formula, whereupon the components of the internal tissues of the biological object have been measured.

Further, the biological information measuring apparatus according to Patent Document 1 is provided with a transfer-drive mechanism capable of causing the confocal optical system, and the biological object to be relatively and three-dimensionally transferred. In Patent Document 1, there has been proposed the biological information measuring apparatus in which the focal position of the confocal optical system is relatively and three-dimensionally transferred against the biological object by relatively and three-dimensionally transferring the confocal optical system, and the biological object with the use of the transfer-drive mechanism, thereby acquiring three-dimensional data on the internal tissues of a biological object, and reliably identifying a site of the biological object, to be measured, whereupon components of the biological object, at the site, can be reliably and noninvasively measured.

Fig. 19 is a block diagram of the biological information measuring apparatus described in Patent Document 1.

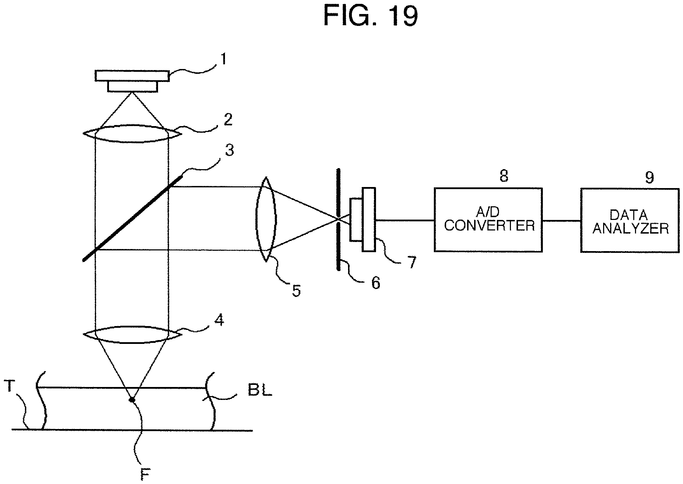

In Fig. 19, a laser beam outputted from a laser diode 1 is shaped into parallel rays by a collimator lens 2 to fall on a half mirror 3 disposed in as-tilted state at an angle of approximately 45° against the optical axis of the collimator lens 2. For the laser diode 1, use is made of a wavelength tunable laser capable of outputting a laser beam at a wavelength in a range of, for example, 1600 to 1700 nm, where glucose absorption is relatively large.

The parallel rays having transmitted through the half mirror 3 is condensed by an objective lens 4 to be irradiated against the internal tissues of a biological object BL placed on a placement platform T. The laser beam reflected from the internal tissues of the biological object BL falls on the objective lens 4 again to be shaped into parallel rays, and the parallel rays are caused to fall on the half mirror 3, whereupon an optical path thereof is changed such that the parallel rays are reflected in a direction at an angle of approximately 90° against the optical axis of the objective lens 4.

The parallel rays whose optical path has been changed by the half mirror 3 to be thereby reflected are condensed into a laser beam by a lens 5, the laser beam falling on a pinhole 6. The laser beam having passed through the pinhole 6 is caused to fall on a photo detector 7 to be subsequently converted into an electric signal.

The photo detector 7 converts the laser beam into the electric signal whose intensity and magnitude will increase or decrease according to luminous energy of the laser beam as received, and the electric signal is delivered to an A/D converter 8. The A/D converter 8 converts the electric signal delivered from the photo detector 7 into digital data to be delivered to a data analyzer 9.

When respective laser beams at not less than two wavelengths differing from each other are irradiated against the biological object BL, the data analyzer 9 executes quantitative analysis of a component of the biological object BL on the basis of a plurality of electric signals as converted and outputted from the photo detector 7.

More specifically, in the case of quantifying a blood glucose value, that is, concentration of glucose in blood, since an analytical curve indicating correlation between the concentration of glucose in a measured blood, and absorbance of a laser beam is stored in the data analyzer 9 beforehand, the data analyzer 9 executes quantification of the concentration of glucose in the blood of the biological object BL on the basis of the analytical curve.

The following Patent document 1 is available as a related art literature related to the conventional biological information measuring apparatus described as above, and the following Patent document 2 is also available as a related art literature related to biological information on a measurement site of a finger.

### [Related Art Literature]

### [Patent Documents]

[Patent Document 1] JP2008-301944A
[Patent Document 2] JP2005-296635A

With the conventional biological information measuring apparatus described as above, however, because the laser diode 1 in use as a light source of the confocal optical system is continuously driven, if continuous measurement is carries out for relatively long time, or output luminous energy of the laser diode 1 is increased in order to enhance an S/N ratio of a detection signal, this will cause an increase in temperature of a measurement site of the biological object BL, thereby raising the risk that a component as a measurement subject, at the measurement site, undergoes alteration in quality, or the measurement site is damaged.

Further, in the case of the conventional biological information measuring apparatus, with respect to the measurement site, no description other than "a biological object, such as an arm, and so forth, is placed on a placement platform" is given, and a specific measurement site, such as, for example, which part of the arm is appropriate, and so forth, is not identified.

Accordingly, there is a possibility that an appropriate and specific measurement site is not necessarily selected by measurement, and there is a risk that measurement results based on a common measurement condition cannot be obtained because the measurement site varies.

Further, it has been described that blood is collected from a subject under test to measure the concentration of glucose in the blood, and at the same time, the laser diode 1 is adjusted to acquire data on an analytical curve indigenous to the subject under test, whereupon a CPU carries out quantification of a biological component of the biological object BL on the basis of the analytical curve, however, how to identify the subject under test has not been described.

In this case, it is required that information necessary for identifying each subject under test is separately acquired prior to those measurements to be inputted, in order to preserve and manage data on an analytical curve and measurement data for every subject under test, and the subject under test must be checked at the time of each measurement that the subject under test is the person in question on the basis of specific information on each subject under test, so that a fairly heavy burden will be forced upon a measurement worker.

Further, most of the reflected light that has passed through the pinhole 6 of the confocal optical system to fall on the photo detector 7 is the reflected light at a position of the internal tissue, identified at a focal position F, while scattered light other than the reflected light is unable to pass through the pinhole 6 and is left as it is without being processed although scattered light contains biological information on the periphery of the measurement site of the biological object BL, so that it can be said that utilization efficiency of output light of the laser diode 1 is not necessarily high.

### SUMMARY OF THE INVENTION

Exemplary embodiments of the present invention address the above disadvantages and other disadvantages not described above. However, the present invention is not required to overcome the disadvantages described above, and thus, an exemplary embodiment of the present invention may not overcome any disadvantages.

According to one or more illustrative aspects of the invention, there is provided a biological information measuring apparatus capable of automatically identifying a subject under test, and executing continuous measurement under a common measurement condition having selected a properly identified site at a high S/N ratio for relatively long hours as necessary, the biological information measuring apparatus being also capable of improving utilization efficiency of the output light of a laser.

According to one or more illustrative aspects of the invention, there is provided a biological information measuring apparatus using a confocal optical system provided with a laser serving as a light source thereof, and the biological information measuring apparatus comprises a laser drive means for effecting AC-driving so as to cause pulsed light to be outputted from the laser.

### BRIEF DESCRIPTION OF THE DRAWINGS

Fig. 1 is a view showing a pattern of output light from the laser diode;
Fig. 2 is a block diagram showing an embodiment of a biological information measuring apparatus according to the invention;
Fig. 3 is a block diagram showing a further embodiment of a biological information measuring apparatus according to the invention;
Fig. 4 is a block diagram showing another embodiment of a biological information measuring apparatus according to the invention;
Fig. 5 is a block diagram showing still another embodiment of a biological information measuring apparatus according to the invention;
Fig. 6 is a block diagram showing more still another embodiment of a biological information measuring apparatus according to the invention;
Fig. 7 is a block diagram showing a still further embodiment of a biological information measuring apparatus according to the invention;
Fig. 8 is a block diagram showing more still further embodiment of a biological information measuring apparatus according to the invention;
Fig. 9 is a block diagram showing another embodiment of a biological information measuring apparatus according to the invention;
Fig. 10 is a block diagram showing still another embodiment of a biological information measuring apparatus according to the invention;
Fig. 11 is a block diagram showing a biological information measuring apparatus according to a further embodiment of the invention;
Fig. 12 is a block diagram showing a biological information measuring apparatus according to a still further embodiment of the invention;
Fig. 13(A) to 13(D) each are a schematic representation showing a specific example of the photo detector 7 for use in the apparatus shown in Fig.12;
Fig. 14(A) to 14(D) each a schematic representation showing another specific example of the photo detector 7 for use in the apparatus shown in Fig.12;
Fig. 15 is a schematic representation showing still another specific example of the photo detector 7 for use in the apparatus shown in Fig.12;
Fig. 16(A) to 16(D) each are a schematic representation showing a further specific example of the photo detector 7 for use in the apparatus shown in Fig.12;
Fig. 17 is a block diagram showing a biological information measuring apparatus according to more still another embodiment of the invention;
Fig. 18(A) and 18(B) each are a schematic representation showing a specific example of a photo detector 21 for use in the apparatus shown in Fig.12; and
Fig. 19 is a block diagram of the biological information measuring apparatus described in Patent Document 1.

### DETAILED DESCRIPTION OF EXEMPLARY EMBODIMENTS

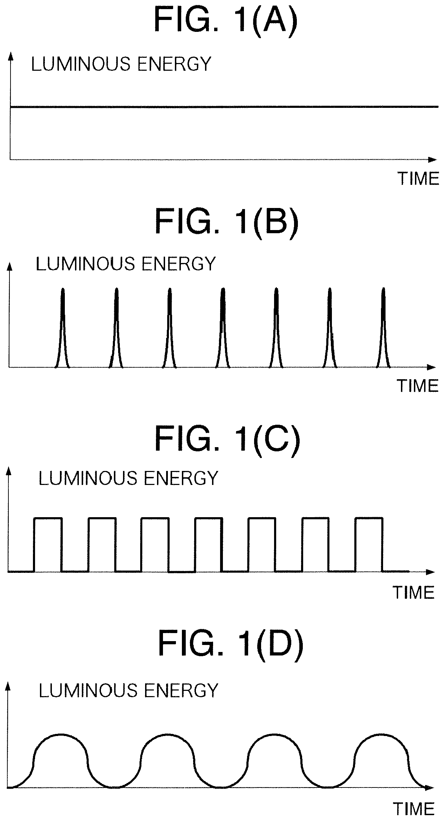

Embodiments of the invention are described hereinafter with reference to the accompanying drawings. Fig. 1 is a view showing a pattern of output light from the laser diode 1 of the biological information measuring apparatus shown in Fig. 19, wherein Fig. 1(A) shows a conventional pattern, and Fig. 1(B) to 1(D) each show a pattern according to one embodiment of the invention.

The laser diode 1 of the conventional confocal optical system has been driven in such a continuous light-emitting state as shown in Fig. 1(A) referred to previously. In contrast, the laser diode 1 according to the invention is AC driven so as to be in such pulsed light-emitting states as shown in Fig. 1(B) to 1(D), respectively. More specifically, with an embodiment shown in Fig. 1(B), the laser diode 1 according to the invention is driven so as to output protruding pulsed light narrow in width, with an embodiment shown in Fig. 1(C), the laser diode 1 is driven so as to output pulsed light rectangular in shape, with duty at 50%, and with an embodiment shown in Fig. 1(D), the laser diode 1 is driven so as to output AC pulsed light semicircular in shape, alternately repeated on the plus side, and the minus side over time.

If the laser diode 1 is AC driven so as to be in the pulsed light-emitting states shown in Fig. 1(B) to 1(D), respectively, thereby increasing output luminous energy of the laser diode 1 in order to execute continuous measurement for many hours, and to enhance the S/N ratio of the detection signal, this will cause an amount of increase in energy given to a measurement site of the biological object BL to be significantly reduced as compared with the case of driving the laser diode 1 in the continuous light-emitting state as shown in Fig. 1(A). More specifically, with the embodiments shown in Fig. 1 (C), and 1 (D), respectively, a maximum increase in the energy is reduced by 1/2 of that in the example shown in Fig. 1(A) while with the embodiment, shown in Fig. 1(B), an increase in the energy is reduced by 1/10 or more as compared with the example shown in Fig. 1(A).

Thus, even if the laser diode 1 is AC driven so as to be in the pulsed light-emitting states shown in Fig. 1(B) to 1(D), respectively, and the output luminous energy of the laser diode 1 is increased in order to execute the continuous measurement for many hours, and to enhance the S/N ratio of the detection signal, it is possible to implement a biological information measuring apparatus capable of executing a stable measuring operation without causing alteration in quality of a component as a measurement subject, at the measurement site of the biological object BL, and without damaging the measurement site.

The AC driving of the laser diode 1, according to the invention, can be applied to not only the biological information measuring apparatus configured as shown in Fig. 19, but also to biological information measuring apparatuses of various configurations shown hereunder, respectively.

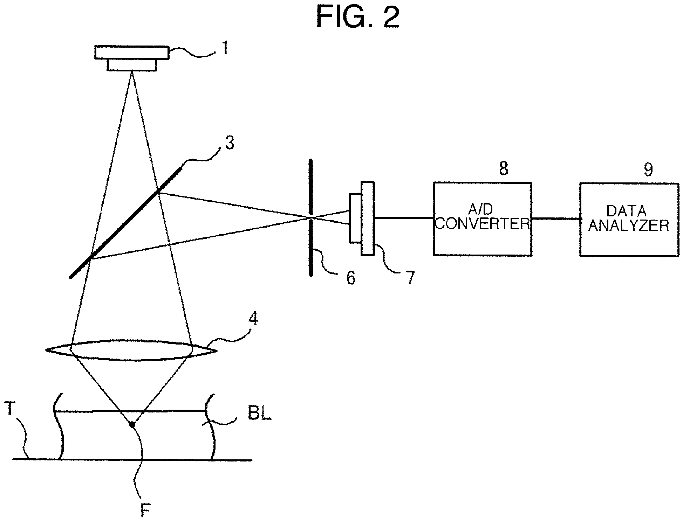

A biological information measuring apparatus shown in Fig. 2 is configured such that output light of the laser diode 1 is condensed by an objective lens 4 without being shaped into parallel rays, and a condensed beam is irradiated against the internal tissues of a biological object BL as a measurement target.

In Fig. 2, a laser beam outputted from the laser diode 1 is caused to fall on a half mirror 3 disposed in a posture having inclination substantially at 45° to the optical axis of the laser diode 1. For the laser diode 1, use is made of a wavelength tunable laser capable of outputting a laser beam in a wavelength range of, for example, 1500 to 1700 nm, where glucose absorption is relatively large. In case one unit of the laser diode is incapable of outputting a laser beam in the wavelength range of 1500 to 1700 nm, use may be made of combination of a plurality of the laser diodes.

The laser beam having transmitted through the half mirror 3 is condensed by an objective lens 4 to be used for irradiation of the internal tissues of the biological object BL. Those elements including the laser diode 1, the half mirror 3, and the objective lens 4 make up an irradiation system for irradiating the internal tissues of the biological object BL, the irradiation system being disposed such that the respective optical axes thereof are opposed to the biological object BL

The laser beam reflected by the internal tissues of the biological object BL is caused to fall on the half mirror 3 again via the objective lens 4, whereupon the laser beam has its optical path altered so as to be reflected in a direction substantially at 90° to the optical axis of the laser diode 1. The laser beam reflected by the half mirror 3, the optical path thereof having been altered, is caused to fall directly on a pinhole 6 without traveling through a lens. The laser beam having passed through the pinhole 6 is caused to fall on a photo detector 7 to be subsequently converted into an electric signal.

The photo detector 7 converts the laser beam into an electric signal that varies in intensity and magnitude in accordance with luminous energy of the laser beam as received, thereby inputting the electric signal to an A/D converter 8. The A/D converter 8 converts the electric signal delivered from the photo detector 7 into digital data to be delivered to a data analyzer 9. Those elements including the pinhole 6, and the photo detector 7 make up a light reception system, and the optical axis of the light reception system is disposed in a direction orthogonal to the optical axis of the irradiation system. Further, the laser diode 1, the objective lens 4, the pinhole 6, and the photo detector 7 make up a confocal optical system.

The data analyzer 9 executes quantitative analysis of components of the biological object BL on the basis of a plurality of the electric signals converted and outputted from the photo detector 7 when respective laser beams at not less than two different wavelengths are irradiated against the biological object BL.

With the adoption of such a configuration as described, the lens 2, and the lens 5, provided in the case of the conventional biological information measuring apparatus shown in Fig. 19, are no longer required, so that assembly adjustment, such as optical axis alignment among various optical parts, and so forth, can be simplified as compared with the case of the conventional biological information measuring apparatus.

Further, the laser beam outputted from the laser diode 1 is condensed by the objective lens 4 without being shaped into parallel rays to be used for irradiation of a measurement site inside a biological object, so that it is possible to precisely fetch measurement data on a desired measurement site in the biological object BL.

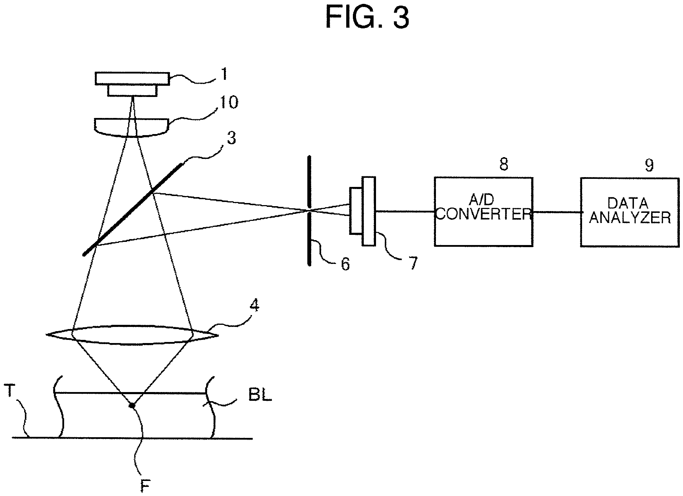

Fig. 3 is a block diagram showing another embodiment of a biological information measuring apparatus according to the invention. With the biological information measuring apparatus shown in Fig. 3, the flux of a laser beam outputted from the laser diode 1 is expanded by use of a planoconvex lens 10. By so doing, an optical path length can be shortened, and the biological information measuring apparatus can be reduced in size by the extent that the optical path length is shortened.

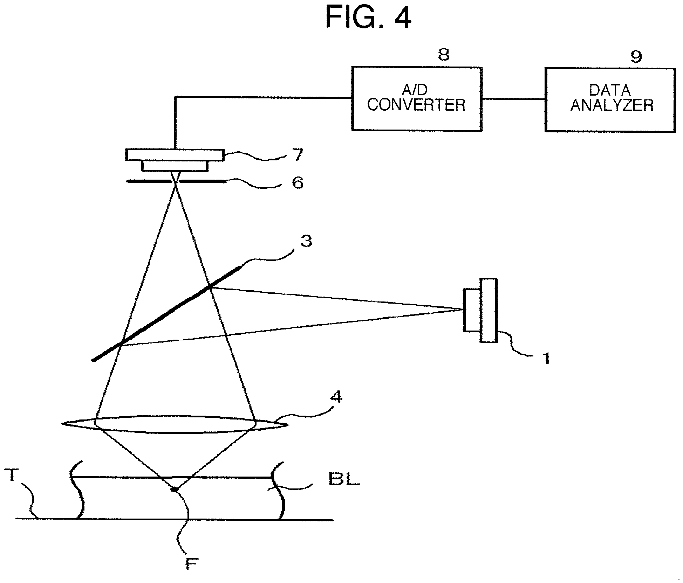

Fig. 4 is also a block diagram showing still another embodiment of a biological information measuring apparatus according to the invention. In the case of the embodiment shown in Fig. 4, a light reception system comprised of a pinhole 6, and a photo detector 7 is disposed in such a way as to oppose a biological object BL while a laser diode 1 is disposed in a direction orthogonal to the optical axis of the light reception system. By so doing, component measurement similar to that shown in Fig. 2 can be carried out.

With a configuration shown in Fig. 4, if a planoconvex lens 10 identical to that shown in Fig. 3 is provided not only in the front of a laser diode 1, but also between a pinhole 6 on a photo detector 7 side of the light reception system, and a half mirror 3, the flux of a laser beam can be expanded, so that an optical path length can be shortened, thereby miniaturizing the biological information measuring apparatus.

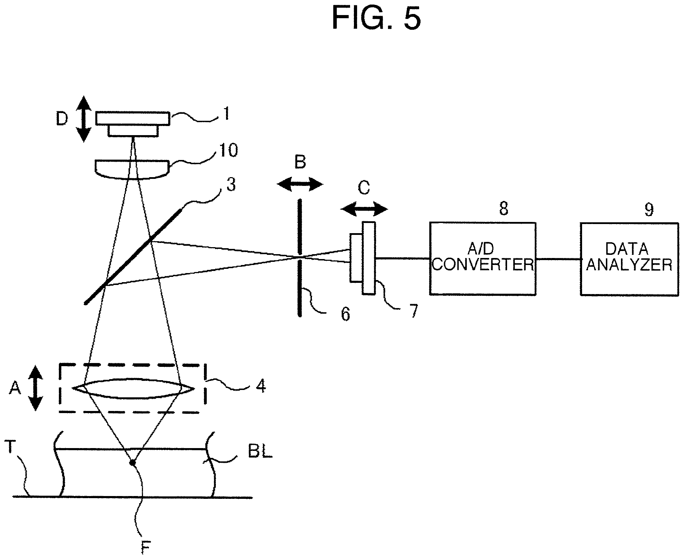

Fig. 5 is also a block diagram showing a further embodiment of a biological information measuring apparatus according to the invention. The biological information measuring apparatus shown in Fig. 5 is made up such that an objective lens 4 is movable along the direction of an optical axis A. By so doing, an image location in a biological object BL can be moved in the depth-wise direction.

Further, in combination with movement of objective lens 4, a pinhole 6 may be moved along the direction of an optical axis B, and a photo detector 7 may be moved along the direction of an optical axis C.

With such a configuration as shown in Fig. 5, by incorporating a mechanism for causing the biological information measuring apparatus comprised of an irradiation system, and a light reception system to be relatively moved against a biological object BL, in an x-axis direction and a y-axis direction, respectively, it is possible to acquire three-dimensional information on the interior of the biological object BL.

Further, with the biological information measuring apparatus shown in Fig. 5, the apparatus is made up such that the objective lens 4 is movable along the direction of the optical axis A, however, alternatively, the objective lens 4 may be fixed, and a laser diode 1 is rendered movable along the direction of an optical axis D while a pinhole 6, and a photo detector 7 are rendered movable while synchronized with movement of the laser diode 1 in the respective directions of an optical axes B, C.

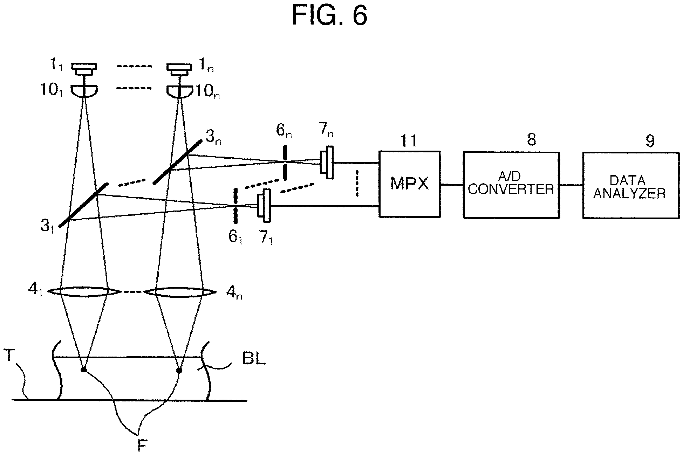

Fig. 6 is also a block diagram showing a still further embodiment of a biological information measuring apparatus according to the invention. With the biological information measuring apparatus shown in Fig. 6, the biological information measuring apparatus of a configuration shown in Fig. 3 is formed as a unit, and a plurality (n-pieces) of the units lined up in an array to be integrated together. Because the respective units for measurement are arranged in an array structure, a set of signals at respective wavelengths differing from each other, sent out from different focal positions in a biological object BL can be acquired by one operation. Further, output signals from respective photo detectors 7 are inputted to a common A/D converter 8 via a multiplexer 11 to be converted into digital data. However, if processing at a higher speed is required, the respective photo detectors 7 may be provided with a dedicated A/D converter.

With each of the biological information measuring apparatus described in the foregoing, if the laser diode 1 is driven by use of an automatic output control loop such that intensity of light from the laser diode 1, irradiated against a measurement target, is maintained at a predetermined value, stable measurement can be carried out.

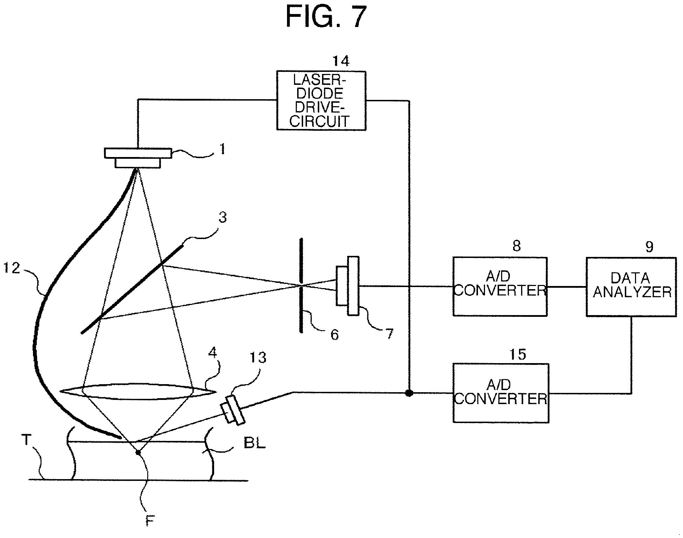

Fig. 7 is a block diagram showing an embodiment wherein such a configuration as described is applied to the biological information measuring apparatus shown in Fig. 2. With the biological information measuring apparatus shown in Fig. 7, a portion of output light of a laser diode 1 is caused to irradiate against the surface of a biological object BL as a measurement target, via an optical fiber 12, reflected light from the surface of biological object BL is detected by a second photo detector 13, and an output signal of the second photo detector 13 is delivered to a laser-diode drive-circuit 14, whereupon the laser-diode drive-circuit 14 drives the laser diode 1 in such a way as to keep intensity of the output light of the laser diode 1 at a predetermined value.

By so doing, it is possible to inhibit variation in intensity of output light, attributable to temperature variation of the laser diode 1, and spatial intensity variation thereof, so that stable component-measurement results can be obtained.

Further, the output signal of the second photo detector 13 is added to a data analyzer 9 via an A/D converter 15, whereupon an output signal of a first photo detector 7 is divided by the output signal of the second photo detector 13, and the result is standardized, thereby enabling compensation for variation due to variation in the output of the laser diode 1, and variation due to reflection at the surface of the biological object BL as the measurement target.

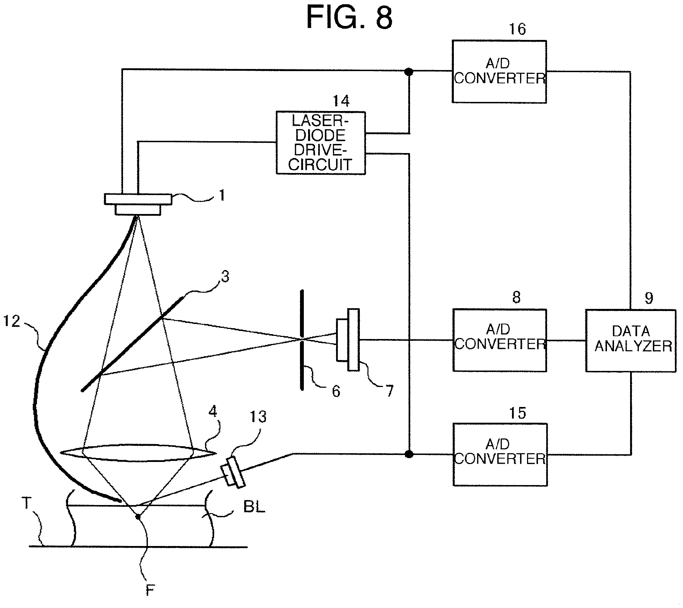

Fig. 8 is a block diagram showing another embodiment of a biological information measuring apparatus according to the invention, wherein use is made of the laser diode 1 of Fig. 7, incorporating a third photo detector (not shown) for monitoring the output light of the laser diode 1. An output signal of the third photo detector is also delivered to the laser-diode drive-circuit 14, whereupon the laser-diode drive-circuit 14 drives the laser diode 1 in such a way as to keep intensity of the output light of the laser diode 1 at the predetermined value.

The output signal of the third photo detector is also delivered to the data analyzer 9 via an A/D converter 16, whereupon the output signal of the first photo detector 7 is divided by the output signal of the third photo detector, and the result is standardized. As a result, the data analyzer 9 executes linear combination of two standardized signals to find combination coefficients by multivariate analysis, thereby compensating, with high precision, for the variation due to output variation of the laser diode 1, and the variation due to reflection at the surface of the biological object BL, on the basis of these values.

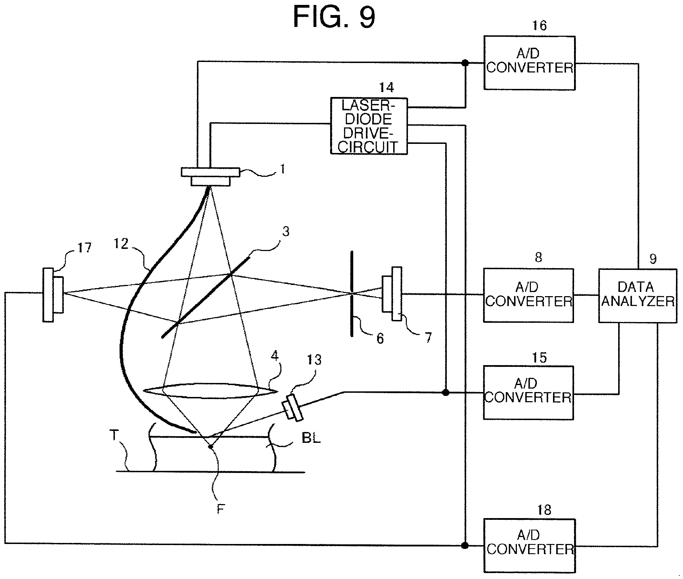

Fig. 9 is a block diagram showing a biological information measuring apparatus according to still another embodiment of the invention, wherein a fourth photo detector 17 for detecting reflection light reflected by a half mirror 3, and an A/D converter 18 for converting an output signal of the fourth photo detector 17 into a digital signal are added to the embodiment shown in Fig. 8. The fourth photo detector 17 detects spatial variation of output light of a laser diode 1. The output signal of the fourth photo detector 17 is also delivered to a laser-diode drive-circuit 14, whereupon the laser-diode drive-circuit 14 drives the laser diode 1 in such a way as to keep intensity of the output light of the laser diode 1 at the predetermined value.

Further, the output signal of the fourth photo detector 17 is also delivered to the data analyzer 9 via an A/D converter 18, whereupon the output signal of the first photo detector 7 is divided by the output signal of the fourth photo detector 17, and the result is standardized. As a result, the data analyzer 9 executes linear combination of three standardized signals to find combination coefficients by multivariate analysis, thereby compensating, with higher precision, for the variation due to output variation of the laser diode 1, spatial variation thereof, and the variation due to reflection at the surface of the biological object BL, on the basis of these values.

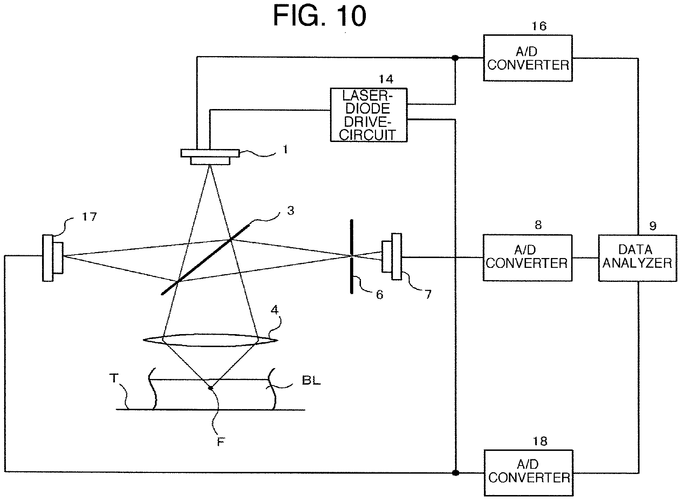

Fig. 10 is a block diagram showing a biological information measuring apparatus made up by omitting a signal system comprising the optical fiber 12, the second photo detector 13, and the A/D converter 15 out of the biological information measuring apparatus shown in Fig. 9. With such a configuration as shown in Fig. 10, a laser-diode drive-circuit 14 drives a laser diode 1 in such a way as to keep intensity of the output light of the laser diode 1 at the predetermined value on the basis of respective output signals of a third photo detector, and a fourth photo detector 17. Then, a data analyzer 9 executes compensation, with high precision, for output variation of the laser diode 1, and spatial variation of the output light thereof, on the basis of a standardized signal obtained by dividing the output signal of the first photo detector 7 by the output signal of the third photo detector, and a standardized signal obtained by dividing the output signal of the first photo detector 7 by the output signal of the fourth photo detector 17.

With the biological information measuring apparatuses shown in Fig. 9, 10, respectively, for the laser diode 1, use is made of a laser diode incorporating the third photo detector for monitoring the output light of the laser diode 1, however, in accordance with compensation accuracy required of the apparatus, a laser diode without the third photo detector incorporated therein may be used as is the case with the biological information measuring apparatus shown in Fig. 6.

Now, such configurations as shown in Fig. 7 to 10, respectively, can be applied to not only the biological information measuring apparatus shown in Fig. 2, but also any of the biological information measuring apparatuses shown in Fig. 3 to 6.

With each of the biological information measuring apparatuses described in the foregoing, there has been shown an example in which a fixed focus lens is used as the objective lens 4, however, use may be made of a variable focus lens. For the variable focus lens, use can be made of a liquid crystal lens constructed by sealing liquid crystals in a space resembling a lens in shape to thereby cause a change of apparent refractive index of the liquid crystals by adjusting a voltage applied thereto. With the liquid crystal lens, a change in refractive index of a constituent material thereof will cause a change in focal length thereof although the shape of the liquid crystal lens remains unchanged.

If the variable focus lens described as above is used as the objective lens 4, a measurement position in the depth-wise direction within the internal tissues of the biological object BL can be suitably set by adjusting a voltage applied to the variable focus lens without moving a position thereof in the direction of the optical axis, thereby simplifying a lens-transfer mechanism.

With each of the biological information measuring apparatuses described in the foregoing, there has been shown an example in which the wavelength tunable laser is used as the light source, however, if a measurement component has been identified, use may be made of a single wavelength laser.

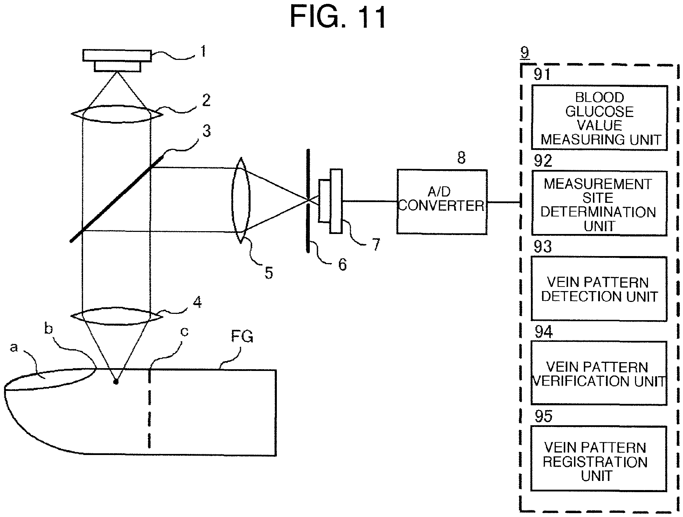

Fig. 11 is also a block diagram showing a biological information measuring apparatus according to a further embodiment of the invention, in which parts in common with those in Fig. 19 are denoted by like reference numerals. In Fig. 11, a biological object BL is a finger FG, and a confocal optical system identifies a portion of skin, in a range of nail epithelium "b" of the finger FG to the first joint "c" thereof, as a measurement site before measurement.

Meanwhile, a data analyzer 9 is provided with a blood glucose value measuring unit 91, a measurement site determination unit 92, a vein pattern detection unit 93, a vein pattern verification unit 94, a vein pattern registration unit 95, and so forth.

The blood glucose value measuring unit 91 measures a blood glucose value on the basis of a detection signal of a confocal optical system, outputted via an A/D converter 8, as previously described.

The measurement site determination unit 92 identifies the skin of the finger FG, and a nail "a" thereof on the basis of the detection signal of the confocal optical system, outputted via the A/D converter 8, to detect the portion of the skin, in the range of the nail epithelium "b" of the finger FG to the first joint "c" thereof, whereupon the measurement site determination unit 92 visualizes to the effect that a measurement site is appropriate by switching color of a display message in a display (not shown), or display color of a lamp from, for example, red to blue.

Thus, by visualizing the portion of the skin, in the range of the nail epithelium "b" of the finger FG to the first joint "c" thereof, as the appropriate measurement site, on the basis of the detection signal of the confocal optical system, and displaying the same, it is possible to decide, and direct the appropriate measurement site by use of a means in common with measurement of the blood glucose value without preparing a separate means for deciding a measurement site, thereby rendering it possible to prevent variation in the measurement site decided at the time of the measurement of the blood glucose value, and to obtain highly reliable measurement results with few measurement error, based on the common measuring condition.

The vein pattern detection unit 93 detects a vein pattern of the finger FG, as one type of biological information on the basis of the detection signal of the confocal optical system, outputted via the A/D converter 8.

The vein pattern verification unit 94 verifies the vein pattern of the finger FG, detected by the vein pattern detection unit 93, against vein patterns of the respective fingers FG of the known subjects under test registered in the vein pattern registration unit 95 in advance. Thereafter, if the vein patterns match each other, the vein pattern verification unit 94 identifies a subject under test on the basis of the registered data while if data on the matching vein pattern is unavailable, the vein pattern together with personal data of a subject under test, such as the name, date of birth, sex, and so forth, is newly registered in the vein pattern registration unit 95 in preparation of the subsequent verification.

Thus, by detecting a vein pattern of the finger FG on the basis of the detection signal of the confocal optical system before verification, thereby identifying a subject under test, it is possible to automatically identify the subject under test by use of the means in common with measurement of the blood glucose value without preparing a separate means for identifying a subject under test.

As a result, it is possible to automatically preserve, and manage measurement data on blood glucose values on a subject-by-subject basis, and to carry out highly accurate correction on the basis of data on an analytical curve unique to an individual subject under test at the time of the measurement of the blood glucose value where necessary.

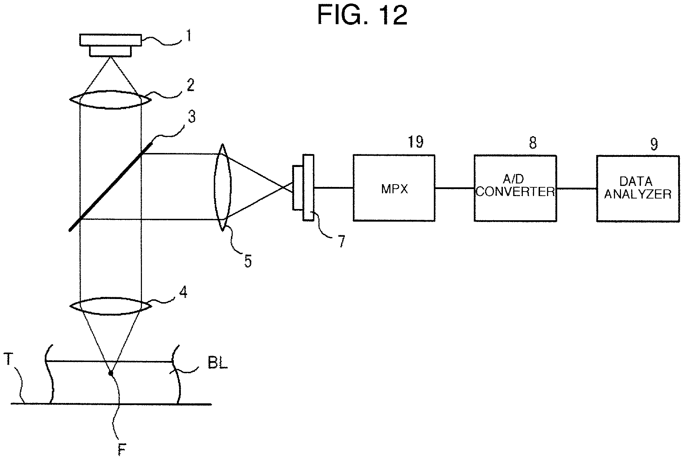

Fig. 12 is also a block diagram showing a biological information measuring apparatus according to a still further embodiment of the invention, in which parts in common with those in Fig. 19 are denoted by like reference numerals. The apparatus of Fig. 12 differs from the apparatus of Fig. 19 in that a pinhole 6 is dispensed with by modifying the structure of a photo detector 7, and an output signal of the photo detector 7 is inputted to an A/D converter 8 via a multiplexer 19.

In Fig. 12, parallel rays having transmitted through a half mirror 3 is condensed by an objective lens 4 to fall on internal tissues of a biological object BL. A laser beam reflected by the internal tissues of the biological object BL falls again on the objective lens 4 to be shaped into parallel rays, the parallel rays falling on the half mirror 3, whereupon an optical path thereof is changed such that the parallel rays are reflected in the direction at an angle of substantially 90° against the optical axis of the objective lens 4.

The parallel rays whose optical path is changed by the half mirror 3 before reflection is condensed into a laser beam by a lens 5 to fall on the photo detector 7 comprised of a plurality of photodiodes to be converted into electric signals,

The photo detector 7 converts the laser beam into the electric signals whose intensity and magnitude will increase or decrease according to luminous energy of a laser beam as received, and delivers output signals of the plurality of photodiodes to the A/D converter 8 via the multiplexer 19. The A/D converter 8 converts the electric signals delivered from the plurality of the photodiodes of the photo detector 7 into digital data to be delivered to a data analyzer 9.

The data analyzer 9 executes quantitative analysis of components of the biological object BL on the basis of the plurality of electric signals that are converted and outputted from the respective photodiodes of the photo detector 7 when the biological object BL is irradiated with the respective laser beams at not less than two wavelengths differing from each other.

With the adoption of a configuration shown in Fig. 12, adjustment in assembling, such as alignment in optical axis between optical parts, can be simplified as compared with the case of a configuration shown in Fig. 19.

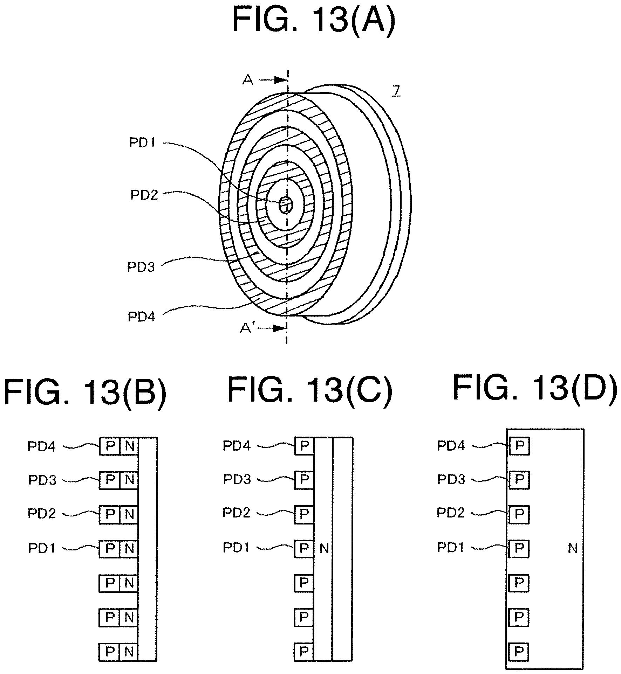

Fig. 13(A) to 13(D) each are a schematic representation showing a specific example of the photo detector 7 for use in the apparatus shown in Fig.12, in which Fig. 13(A) is a perspective view thereof, and Fig. 13(B) to 13(D) each are a sectional view thereof, taken on line A-A' of Fig. 13(A).

In Fig. 13(A) to 13(D), a plurality of photodiodes formed so as to be concentrically circular in shape are provided on a light-receiving surface of the photo detector 7, as shown in Fig. 13(A). With the present embodiment, there is shown an example in which four pieces of the photodiodes PD 1 to PD4 are provided, however, it is to be pointed out that the number of photodiode patterns that are concentrically circular, and a pitch therebetween are not limited to those according to the present embodiment, and that an appropriate specification be selected by taking into consideration a semiconductor manufacturing process, analytic processing capacity required of the apparatus, and so forth.

As a sectional configuration of the photo detector 7 shown in Fig. 13(A), there are conceivable a case where photodiode patterns are formed by depositing an n-type diffusion layer, and a p-type diffusion layer in that order on a substrate, the photodiode patterns being physically separated from each other in such a way as to be concentrically circular in shape, as shown in Fig. 13(B), another case where photodiode patterns are formed by depositing a p-type diffusion layer on an n-type diffusion layer formed across the surface of a substrate, the photodiode patterns being physically separated from each other in such a way as to be concentrically circular in shape, as shown in Fig. 13(C), and a still another case where photodiode patterns are formed by depositing a p-type diffusion layer in the vicinity of the surface of an n-type diffusion substrate, the photodiode patterns being physically separated from each other in such a way as to be concentrically circular in shape, as shown in Fig. 13(D).

With the use of the photo detector 7 provided with the plurality of the photodiodes PD 1 to PD4, concentrically circular in shape, as show in Fig. 13(A), the respective photodiodes PD1 to PD4 of the photo detector 7 detect luminous energy according to an imaging pattern condensed by the lens 5.

That is, with the use of the photo detector 7 shown in Fig. 13(A), the photo detector 7 can detect only scattered signals from the focal position of the objective lens 4, as intended, by measuring signal intensities of the respective photodiodes PD1 to PD4, concentrically circular in shape, to thereby integrate a signal of the photodiode PD1, large in intensity, even if the photo detector 7 is not at a confocal imaging position.

Further, scattering signals from other than the focal position of the objective lens 4 are obtained as respective signals from the photodiodes PD2 to PD4, positioned on outer peripheries outside the photodiode PD1 capturing a signal large in intensity, and these scattering signals can be utilized for detection of biological information such as information on the surface of a skin, a vein pattern of a finger, and so forth.

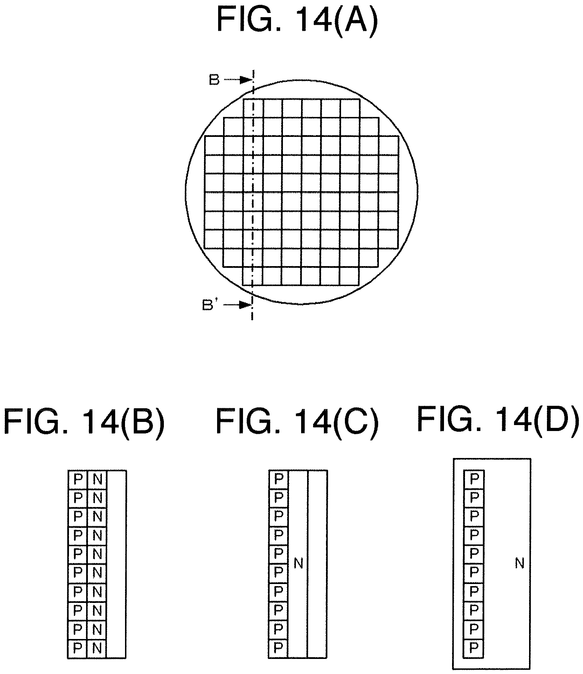

Fig. 14(A) to 14(D) each are a schematic representation showing another specific example of the photo detector 7 for use in the apparatus shown in Fig.12, in which Fig. 14(A) is a perspective view thereof, and Fig. 14(B) to 14(D) each are a sectional view thereof, taken on line B-B' of Fig. 14(A).

In Fig. 14(A) to 14(D), a plurality of photodiodes disposed in a matrix fashion are provided throughout a light-receiving surface of the photo detector 7, as shown in Fig. 14(A).

It is conceivable that a sectional configuration of the photo detector 7 shown in Fig. 14(A) includes a case where a deposition pattern of a p-type diffusion layer, and an n-type diffusion layer formed on a substrate is in the matrix fashion, as shown in Fig. 14(B), another case where a p-type diffusion layer patterned in the matrix fashion is provided on an n-type diffusion layer formed throughout the surface of a substrate, as shown in Fig. 14(C), and a still another case where a p-type diffusion layer patterned in the matrix fashion is provided in the vicinity of the surface of an n-type diffusion substrate, as shown in Fig. 14(D).

With the use of the photo detector 7 shown in Fig. 14(A), even if the photo detector 7 is not at a confocal imaging position, and aberration occurs to an optical system, the photo detector 7 can detect only scattering signals from a focal position of the objective lens 4, as intended, by selecting a signal of the photodiode, large in intensity, to thereby integrate the signal. Further, scattering signals from other than the focal position of the objective lens 4 are obtained as signals from photodiodes other than the photodiode capturing a detection signal large in intensity, and these signals can be utilized for detection of biological information such as information on the surface of a skin, a vein pattern of a finger, and so forth.

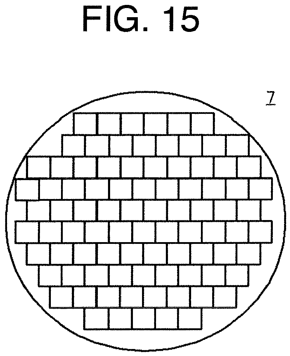

Fig. 15 is also a schematic representation showing still another specific example of the photo detector 7 for use in the apparatus shown in Fig.12, and in this example, an array pattern of the photodiodes in Fig. 14(A), in the vertical direction, is shifted by a 1/2 pitch in the transverse direction. With the use of the photo detector 7 arrayed as above, it is possible to disperse and mitigate effects of dead bands against detection of an optical signal, present as a regular grid-like matrix pattern between the photodiodes adjacent to each other in the case of the photo detector 7 of Fig. 14(A).

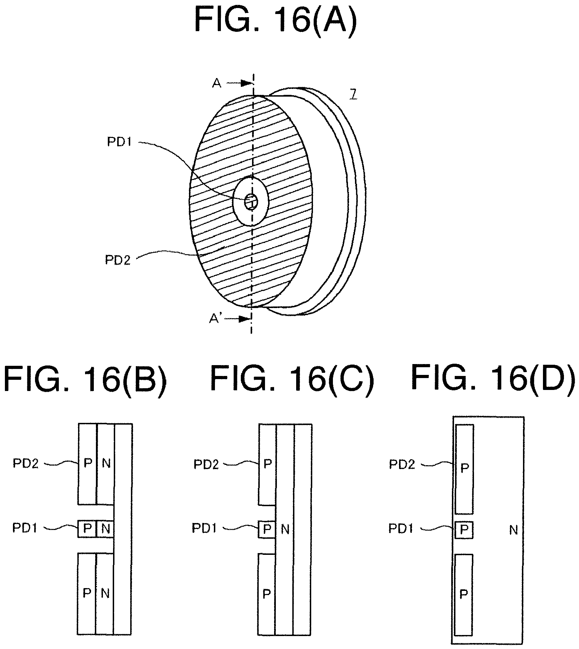

Fig. 16(A) to 16(D) each are also a schematic representation showing a further specific example of the photo detector 7 for use in the apparatus shown in Fig.12, and in this example, a photodiode PD1 in the central region of the photo detector 7, and a photodiode PD2 in the outer peripheral region of the photodiode PD1 are provided so as to be electrically isolated from each other.

It is conceivable that a sectional configuration of the photo detector 7 shown in Fig. 16(A) includes a case where photodiode patterns formed by depositing a p-type diffusion layer on an n-type diffusion layer are physically separated from each other over a substrate, as shown in Fig. 16(B), another case where p-type diffusion layers are physically separated from each other on an n-type diffusion layer that is formed throughout the surface of a substrate, as shown in Fig. 16(C), and a still another case where p-type diffusion layers physically separated from each other are formed in the vicinity of the surface of an n-type diffusion substrate, as shown in Fig. 16(D).

With the use of the photo detector 7 shown in Fig. 16(A) to 16(D), the photodiode PD1 in the central region of the photo detector 7, and the photodiode PD 2 in the outer peripheral region of the photodiode PD1 are provided so as to be electrically isolated from each other, so that the respective photodiodes PD1, PD2 of the photo detector 7, detect luminous energy according to an imaging pattern condensed by the lens 5.

More specifically, with the use of the photo detectors 7 shown in Fig. 16(B) to 16(D), respectively, the photodiode PD1 in the central region can receive only scattered light from the focal position of the objective lens 4, and the photodiode PD2 in the outer peripheral region of the photodiode PD1 can receive only scattered light from other than the focal position of the objective lens 4.

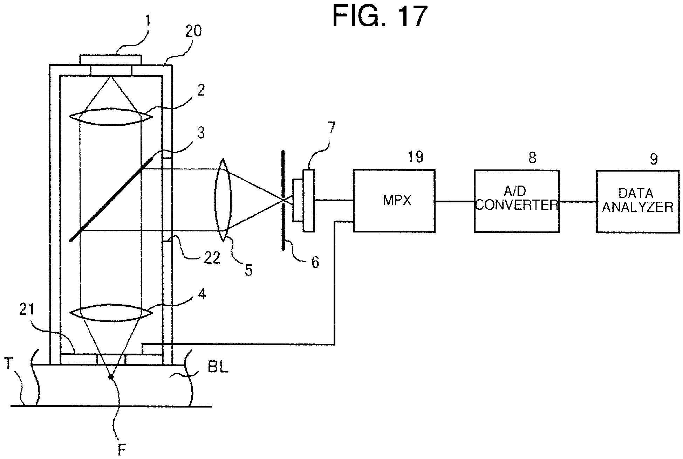

Fig. 17 is a block diagram showing a biological information measuring apparatus according to another embodiment of the invention, in which parts in common with those in Fig. 19 are denoted by like reference numerals. The apparatus of Fig. 17 differs from the apparatus of Fig. 19 in that a laser diode 1, a collimator lens 2, a half mirror 3, and an objective lens 4, making up a confocal optical system, are assembled in a housing 20 cylindrical in shape to be integrated with one end thereof, and the housing 20 is formed so as to enable the other end thereof to come into contact with a measurement region on the surface of a biological object BL.

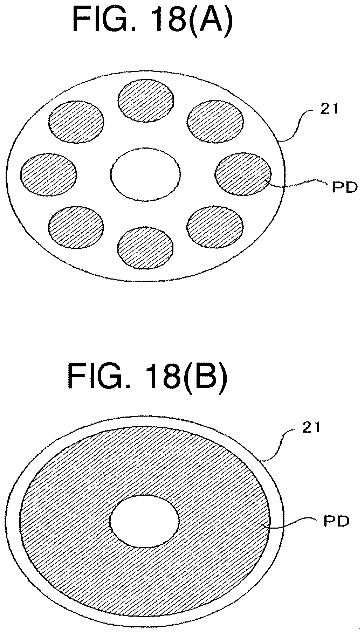

The laser diode 1 is attached to the one end of the housing 20, and a photo detector 21 formed in the shape of a circle as shown in Fig. 18 is attached to the other end of the housing 20, coming into contact with the measurement region on the surface of the biological object BL. Further, a window 22 for allowing a laser beam whose optical path is changed by the half mirror 3 before reflection to outgo therethrough is provided in a side face of the housing 20.

In Fig. 18(A), 18(B), an opening for allowing reflected light from a focal position in the biological object BL to pass therethrough is provided in the central part of the photo detector 21, and 8 pieces of photodiodes PDs for detection of laser beams scattered in the internal tissues of the biological object BL are provided at equal angular intervals in the circumferential direction of the opening at the center. In Fig. 18(B), the opening for allowing the reflected light from the focal position in the biological object BL to pass therethrough is provided in the central part of the photo detector 21, and a PD for detection of laser beams scattered in the internal tissues of the biological object BL is provided on the whole surface of the photo detector 21, around the opening at the center thereof.

In the biological information measuring apparatus of a configuration shown in Fig. 17, a laser beam outgoing from the laser diode 1 is collimated by the collimator lens 2 to subsequently pass through the half mirror 3, and the objective lens 4 before reaching the biological object BL. The laser beam that is scattered and reflected in the biological object BL passes through the objective lens 4, and the half mirror 3 again to be condensed by the lens 5 before passing through a pinhole 6, and falling on a photo detector 7. The pinhole 6 can be omitted where appropriate. An output signal from the photo detector 7 is delivered to an A/D converter 8 via a multiplexer 19. Output data from the A/D converter 8 is delivered to a data analyzer 9, whereupon a predetermined signal-processing is executed.

In the case where the photo detector 21 is as shown in Fig. 18 (A), the laser beam outgoing from the objective lens 4 passes through the opening of the photo detector 21 to fall on the interior of biological object BL before focused. The 8 pieces of the photodiodes PDs provided on the outer periphery of the opening receive substantially all light beans scattered in the interior of biological object BL. Respective output signals from these PDs are delivered to the A/D converter 8 via the multiplexer 19 to be converted into digital signals, and output data of the A/D converter 8 is delivered to the data analyzer 9, whereupon the predetermined signal-processing is executed.

In the case where the photo detector 21 is as shown in Fig. 18 (B), the end face of the housing 20, coming into contact with the measurement region on the surface of the biological object BL, will serve as a photodiode PD large in diameter, having a hole at the center thereof. A laser beam outgoing from the opening at the center of the photo detector 21 falls on the interior of biological object BL to be focused. The photodiode PD large in diameter receives substantially all light beans scattered in the interior of biological object BL. An output signal from the photodiode PD is delivered to the A/D converter 8 via the multiplexer 19 to be converted into a digital signal, and the output data of the A/D converter 8 is delivered to the data analyzer 9, whereupon the predetermined signal-processing is executed.

Further, if a confocal optical system is provided with a mechanism capable of scanning in an x-axis direction, and a y-axis direction, respectively (not shown in Fig. 17), it is possible to obtain three-dimensional information on the interior of biological object BL.

Further, if the confocal optical system is configured so as to be tunable in a z-axis direction as well, this will render it possible to measure scattered and reflected light beams at wavelengths differing from each other (for example, at a wavelength for absorbing a target substance, and a wavelength less absorbing) by use of the same optical system, thereby measuring a blood glucose value on the basis of an analytical curve of glucose absorption.

With the respective biological information measuring apparatuses described in the foregoing, there has been described the case of measuring a blood glucose value in the blood of a human body, however, those biological information measuring apparatuses are also effective for quantitative measurement of blood components other than the blood glucose value, and tissue fluid components.

With those biological information measuring apparatuses, a measurement subject is not limited to the human body, and they are also effective for quantitative measurement of internal substances of an animal, a plant, and so forth, respectively.

Further, a measurement subject is not limited to a biological object, and they are also effective for non-destructive inspection of structure / composition of an agricultural product, a fishery product, a food product, an organic material, and so forth, and quantitative measurement of a chemical substance contained therein.

As described in the foregoing, with the present invention, it is possible to realize a biological information measuring apparatus capable of making adjustment in assembling with relative ease, and precisely fetching measurement data on a desired measurement site of a measurement subject, and the biological information measuring apparatus is suitable for measurement of various components including a blood glucose value in the blood of a human body, and so forth.

With the adoption of such a configuration as described in the foregoing, it is possible to automatically identify a subject under test, and to execute continuous measurement under a common measurement condition having selected a properly identified site at a high S/N ratio for relatively long hours as necessary, and utilization efficiency of the output light of a laser can be improved.

While the present invention has been shown and described with reference to certain exemplary embodiments thereof, other implementations are within the scope of the claims. It will be understood by those skilled in the art that various changes in form and details may be made therein without departing from the spirit and scope of the invention as defined by the appended claims.

## Claims

1. A biological information measuring apparatus using a confocal optical system provided with a laser serving as a light source thereof, said biological information measuring apparatus comprising a laser drive means (14) for effecting AC-driving so as to cause pulsed light to be outputted from the laser.

2. The biological information measuring apparatus according to claim 1, wherein the skin of a finger (FG) is discriminated from a nail (a) thereof on the basis of a detection signal of the confocal optical system, and a portion of the skin, in a range of the nail epithelium (b) of the finger (FG) to the first joint (c) thereof, is indicated as a measurement region.

3. The biological information measuring apparatus according to claim 1, wherein a vein pattern of the finger (FG) is discriminated on the basis of a detection signal of the confocal optical system, thereby identifying a subject under test

4. The biological information measuring apparatus according to claim 1, wherein a photo detector (7, 13, 17) for receiving reflected light from a confocal position of the confocal optical system, and a photo detector (21) for receiving reflected light from the periphery of the confocal position are provided.
